# EUROPEAN PATENT APPLICATION

(11) **EP 4 029 575 A1**
(43) Date of publication of application: **20.07.2022**
(21) Application number: 20863603.5
(22) Date of filing: 27.08.2020
(51) Int. Cl.: A63B 69/00, G06F 3/01, G06F 3/0481

(54) **INFORMATION PROCESSING DEVICE, INFORMATION PROCESSING METHOD, AND PROGRAM**

(30) Priority: 10.09.2019 JP 2019164448
(71) Applicant: Sony Group Corporation, Tokyo 108-0075 (JP)
(72) Inventor: KATSU Masanori, Tokyo 108-0075 (JP)
(74) Representative: MFG Patentanwälte Meyer-Wildhagen Meggle-Freund Gerhard PartG mbB
(86) International application number: PCT/JP2020/032284
(87) International publication number: WO 2021/049304

(57) **Abstract**

The present disclosure relates to an information processing apparatus, an information processing method, and a program capable of facilitating production of load data for providing a more realistic experience.

A data control unit associates relative load data, which is time-series load data relative to a living body, with each temporal section of content. The relative load data is converted into absolute load data, which is time-series load data absolute to a player who reproduces the content, on the basis of a normalization parameter of the player. The technology according to the present disclosure is applicable to a device that creates/edits the relative load data, for example.

## Description

### TECHNICAL FIELD

The present disclosure relates to an information processing apparatus, an information processing method, and a program, and more particularly, to an information processing apparatus, an information processing method, and a program capable of facilitating creation/editing of load data for providing a more realistic experience.

### BACKGROUND ART

In order to provide a user with a more realistic experience, for example, there has been known a technique of recording vital data of an athlete.

Patent Document 1 discloses an information processing apparatus that performs display to prompt a user to perform an operation of reproducing content associated with biological information and causes the biological information and the content to be reproduced in response to an operation of the user. With this arrangement, the user is enabled to experience emotions of another user at the time of content recording.

### CITATION LIST

### PATENT DOCUMENT

Patent Document 1: Japanese Patent Application Laid-Open No. 2017-211862

### SUMMARY OF THE INVENTION

### PROBLEMS TO BE SOLVED BY THE INVENTION

Meanwhile, it has not been easy for a beginner to create or edit load data for providing a load experience corresponding to content because of high difficulty.

The present disclosure has been conceived in view of such a situation, and aims to facilitate creation/editing of load data for providing a more realistic experience.

### SOLUTIONS TO PROBLEMS

An information processing apparatus according to the present disclosure is an information processing apparatus including a data control unit that associates relative load data, which is time-series load data relative to a living body, with each of temporal sections of content, in which the relative load data is converted into absolute load data, which is the time-series load data absolute to a player who reproduces the content, on the basis of a normalization parameter of the player.

An information processing method according to the present disclosure is a method including associating, by an information processing apparatus, relative load data, which is time-series load data relative to a living body, with each of temporal sections of content, in which the relative load data is converted into absolute load data, which is the time-series load data absolute to a player who reproduces the content, on the basis of a normalization parameter of the player.

A program according to the present disclosure is a program for causing a computer to function as a data control unit that associates relative load data, which is time-series load data relative to a living body, with each of temporal sections of content, in which the relative load data is converted into absolute load data, which is the time-series load data absolute to a player who reproduces the content, on the basis of a normalization parameter of the player.

According to the present disclosure, relative load data, which is time-series load data relative to a living body, is associated with each of temporal sections of content, and the relative load data is converted into absolute load data, which is the time-series load data absolute to a player who reproduces the content, on the basis of a normalization parameter of the player.

### BRIEF DESCRIPTION OF DRAWINGS

Fig. 1 is a block diagram illustrating an exemplary configuration of an information processing system according to the present disclosure.
Fig. 2 is a diagram for explaining recording of relative load data.
Fig. 3 is a block diagram illustrating an exemplary configuration of a relative load calculation device.
Fig. 4 is a flowchart for explaining a process of calculating the relative load data.
Fig. 5 is a flowchart for explaining a flow of obtaining a normalization parameter.
Fig. 6 is a diagram illustrating exemplary acquisition of the normalization parameter.
Fig. 7 is a block diagram illustrating an exemplary configuration of a relative load creation/editing device.
Fig. 8 is a flowchart for explaining a process of producing the relative load data.
Fig. 9 is a flowchart for explaining a process of editing the relative load data.
Fig. 10 is a diagram illustrating exemplary screen display of the relative load creation/editing device.
Fig. 11 is a diagram for explaining display of a section timeline panel.
Fig. 12 is a diagram for explaining display of a relative load timeline panel.
Fig. 13 is a diagram for explaining display of a content panel.
Fig. 14 is a diagram for explaining display of a load icon panel.
Fig. 15 is a diagram for explaining editing of the relative load data.
Fig. 16 is a diagram illustrating exemplary display of load icons.
Fig. 17 is a flowchart for explaining a process of changing display of the load icon.
Fig. 18 is a block diagram illustrating an exemplary configuration of a relative load reproduction device.
Fig. 19 is a flowchart for explaining a process of reproducing the relative load data.
Fig. 20 is a block diagram illustrating another exemplary configuration of the relative load reproduction device.
Fig. 21 is a flowchart for explaining the process of reproducing the relative load data.
Fig. 22 is a diagram for explaining load adjustment.
Fig. 23 is a diagram illustrating an exemplary load providing device.
Fig. 24 is a block diagram illustrating an exemplary configuration of a normalization parameter calculation device.
Fig. 25 is a block diagram illustrating an exemplary configuration of a data extraction unit.
Fig. 26 is a flowchart for explaining operation of the data extraction unit.
Fig. 27 is a block diagram illustrating an exemplary configuration of a VO2Max estimation unit.
Fig. 28 is a flowchart for explaining operation of the VO2Max estimation unit.
Fig. 29 is a block diagram illustrating another exemplary configuration of the VO2Max estimation unit.
Fig. 30 is a flowchart for explaining the operation of the VO2Max estimation unit.
Fig. 31 is a diagram for explaining acquisition of the normalization parameter before an event.
Fig. 32 is a block diagram illustrating an exemplary configuration of a computer.

### MODE FOR CARRYING OUT THE INVENTION

Hereinafter, a mode for carrying out the present disclosure (hereinafter referred to as an embodiment) will be described. Note that descriptions will be given in the following order.

1. System Configuration
2. Relative Load Data Calculation
3. Relative Load Data Creation/Editing
4. Relative Load Data Reproduction
5. Normalization Parameter Acquisition
6. Exemplary Computer Configuration

### <1. System Configuration>

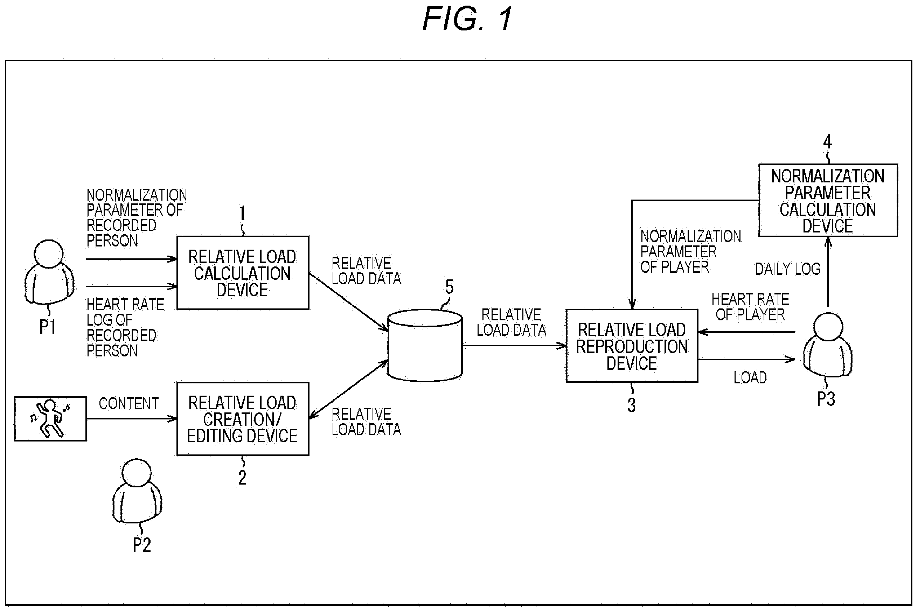

Fig. 1 is a block diagram illustrating an exemplary configuration of an information processing system according to the present disclosure.

In the information processing system of Fig. 1, vital data of a recorded person P1 is recorded, or a creator P2 creates/edits the vital data to provide a user with a more realistic experience. With content associated with such vital data reproduced by a player P3, an experience corresponding to the recorded, created, or edited vital data is provided to the player P3.

The information processing system of Fig. 1 includes a relative load calculation device 1, a relative load creation/editing device 2, a relative load reproduction device 3, and a normalization parameter calculation device 4.

The relative load calculation device 1 calculates relative load data, which is time-series load data relative to a living body, on the basis of a normalization parameter and a heart rate log for the recorded person P1 who is, for example, an athlete. The normalization parameter is a parameter for standardizing (normalizing) a physical load on the recorded person P1, and the heart rate log is time-series data of the heart rate of the recorded person P1.

The relative load data calculated by the relative load calculation device 1 is saved in the data saving unit 5.

The relative load creation/editing device 2 creates/edits the relative load data corresponding to predetermined content in accordance with a creation/editing operation of the creator P2. Specifically, the relative load creation/editing device 2 associates the relative load data created by the creator P2 or the relative load data in which the relative load data saved in the data saving unit 5 is edited by the creator P2 with the predetermined content.

The relative load reproduction device 3 provides a load experience to the player P3 who reproduces the content associated with the relative load data. Specifically, the relative load reproduction device 3 convers the relative load data associated with the content to be reproduced into absolute load data, which is time-series load data absolute to the player P3 on the basis of the normalization parameter of the player P3, and provides it to the player P3. The relative load reproduction device 3 may adjust the absolute load data depending on the heart rate of the player P3 at that time.

The normalization parameter calculation device 4 calculates a normalization parameter for the player P3 on the basis of a daily log including exercise data and biological information in daily life of the player P3.

Hereinafter, a configuration and operation of each of the relative load calculation device 1, the relative load creation/editing device 2, the relative load reproduction device 3, and the normalization parameter calculation device 4 will be described.

### <2. Relative Load Data Calculation>

Conventionally, there are many viewers/listeners who desire the same experience as an athlete to obtain more realistic feeling in sports watching and the like. However, while an athlete has physical strength and can withstand a high load, it has been difficult for a viewer/listener to have the same experience as the athlete.

In view of the above, the relative load calculation device 1 uses not only the vital data of the recorded person such as an athlete but also the normalization parameter of the recorded person, thereby causing the viewer/listener to experience the normalized load.

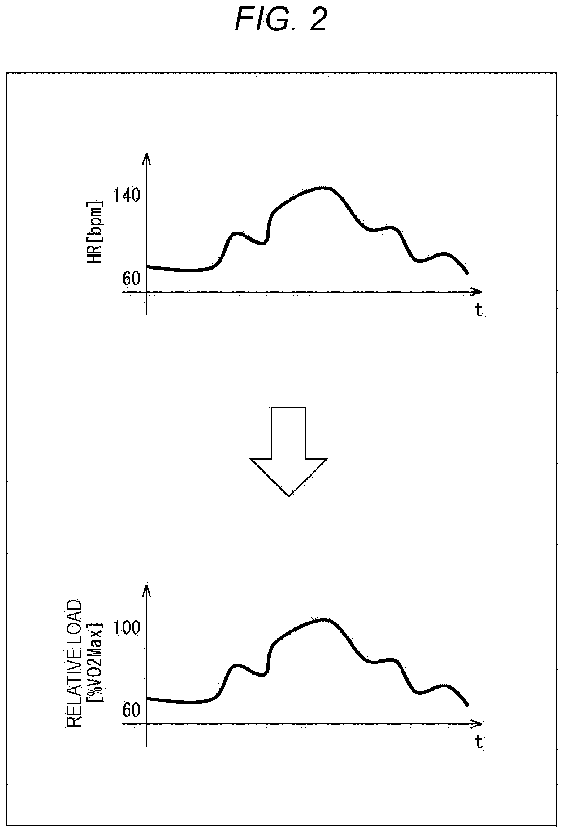

For example, the relative load calculation device 1 normalizes the heart rate (HR) of the recorded person illustrated in the upper part of Fig. 2 with the normalization parameter of the recorded person, thereby calculating the relative load data illustrated in the lower part of Fig. 2. In the present embodiment, a relative value (%VO2Max) of the oxygen intake during a predetermined exercise with respect to the maximum oxygen intake (VO2Max) of the recorded person is calculated as the relative load data.

The maximum oxygen intake represents strength of a cardiopulmonary function, which serves as an indicator of a fitness level. The maximum oxygen intake is measured as an oxygen intake when exercise close to the limit is performed on an aero bike (registered trademark) or the like. While the maximum oxygen intake generally decreases by aging, it has been known that it can be improved by several percent by training. In recent years, it has been proposed to utilize the value of the maximum oxygen intake as a clinical vital sign.

### (Configuration of Relative Load Calculation Device)

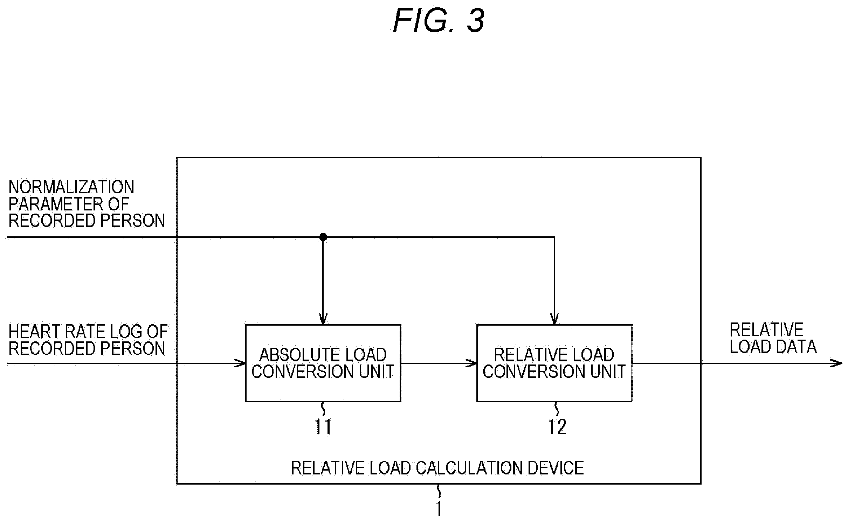

Fig. 3 is a block diagram illustrating an exemplary configuration of the relative load calculation device 1.

The relative load calculation device 1 of Fig. 3 includes an absolute load conversion unit 11 and a relative load conversion unit 12.

The absolute load conversion unit 11 convers the heart rate log of the recorded person into absolute load data on the basis of the normalization parameter of the recorded person, and supplies it to the relative load conversion unit 12.

The relative load conversion unit 12 converts the absolute load data from the absolute load conversion unit 11 into relative load data on the basis of the normalization parameter of the recorded person.

### (Operation of Relative Load Calculation Device)

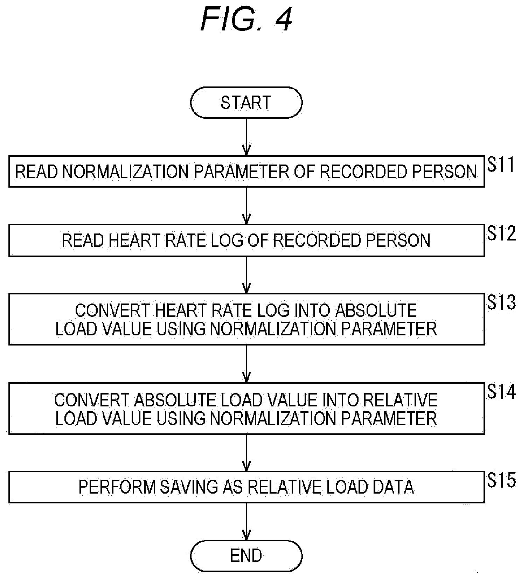

Next, a process of calculating relative load data performed by the relative load calculation device 1 will be described with reference to a flowchart of Fig. 4.

In step S11, the absolute load conversion unit 11 reads the normalization parameter of the recorded person.

In step S12, the absolute load conversion unit 11 reads the heart rate log of the recorded person.

Note that the processing of step S11 and the processing of S12 may be performed in parallel.

In step S13, the absolute load conversion unit 11 converts each value of the heart rate log (bpm) into an oxygen intake (VO2) (ml/min/kg), which is an absolute load value, using the normalization parameter.

In step S14, the relative load conversion unit 12 converts the VO2 (ml/min/kg), which is an absolute load value, into a relative load value (%VO2Max) using the normalization parameter.

In step S15, the relative load conversion unit 12 saves, as relative load data, the time-series data of the converted relative load value in the data saving unit 5.

As described above, the relative load data in which the heart rate log of the recorded person is converted into the relative value (%VO2Max) with respect to the VO2Max of the recorded person is calculated.

### (Exemplary Normalization Parameter Acquisition)

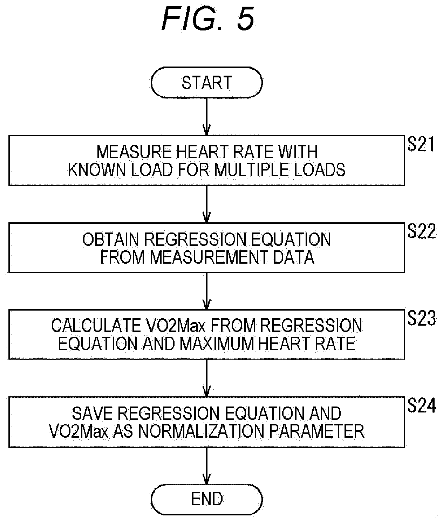

Here, a flow of obtaining the normalization parameter of the recorded person will be described with reference to a flowchart of Fig. 5. The normalization parameter of the recorded person described above is obtained in advance as follows.

In step S21, heart rate measurement with a known load is performed on the recorded person for a plurality of loads.

In step S22, a regression equation is obtained from the obtained measurement data of the heart rate.

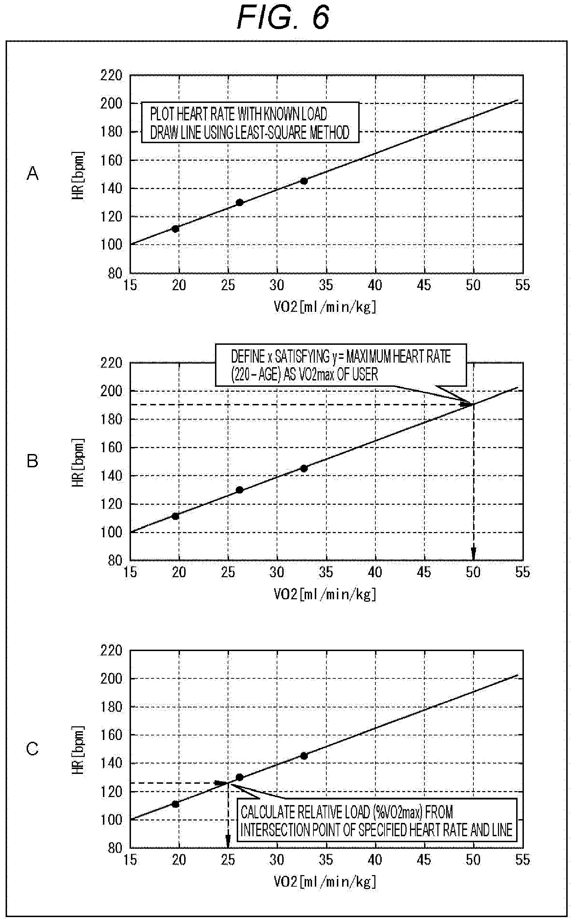

For example, in a case where 110, 130, and 150 (bpm) are obtained as heart rates for values 20, 26, and 33 (ml/min/kg) of three known loads (VO2), a straight line representing a regression equation is obtained by a least-square method on the basis of the obtained three points, as illustrated in A of Fig. 6. In the example of A in the drawing, in a case where the known load is x and the heart rate is y, a regression equation expressed by y = 3.07x + 49.1 is obtained. As expressed in this regression equation, it is known that the load (oxygen intake) is proportional to the heart rate.

In step S23, VO2Max is calculated from the regression equation and a maximum heart rate.

The maximum heart rate can be calculated by 220 - age. In a case where the age of the recorded person is 28 years old, the maximum heart rate of the recorded person is 220 - 28 = 192. In this case, the oxygen intake corresponding to the maximum heart rate, that is, VO2Max, is calculated as x = 50 (ml/min/kg) by the regression equation described above, as illustrated in B of Fig. 6.

In step S24, the regression equation and VO2Max described above are saved as normalization parameters of the recorded person.

The relative load value for the predetermined exercise performed by the recorded person is calculated by the normalization parameters of the recorded person obtained in this manner.

Specifically, in a case where the heart rate of the recorded person at the time performing a certain exercise is 122 (bpm), the load (oxygen intake) of the recorded person is 25 (ml/min/kg), as illustrated in C of Fig. 6. Therefore, the relative load value with respect to VO2Max (50 (ml/min/kg)) of the recorded person is calculated as 50 (%VO2Max).

### <3. Relative Load Data Creation/Editing>

Conventionally, at a time of creating content that allows experience of various loads while viewing the content, it has not been easy for a beginner to create or edit load data for providing the load experience corresponding to the content because of high difficulty.

In view of the above, the relative load creation/editing device 2 provides a user interface (UI) in which load data abstracted by icons are arranged in sections obtained by temporally dividing the content.

### (Configuration of Relative Load Creation/Editing Device)

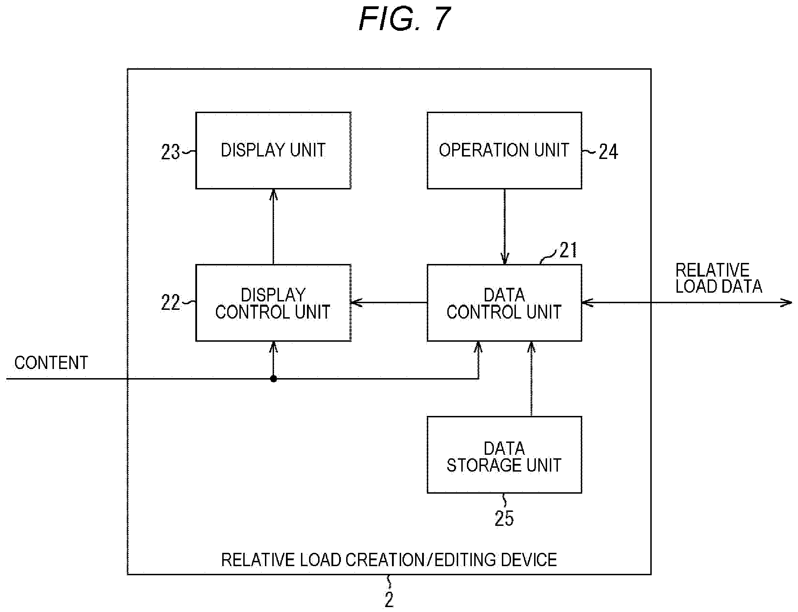

Fig. 7 is a block diagram illustrating an exemplary configuration of the relative load creation/editing device 2.

The relative load creation/editing device 2 is configured as a personal computer (PC), a smartphone, or the like.

The relative load creation/editing device 2 of Fig. 7 includes a data control unit 21, a display control unit 22, a display unit 23, an operation unit 24, and a data storage unit 25.

The data control unit 21 associates time-series load data, which is temporally short relative load data, with each temporal section of the content according to operation information from the operation unit 24, thereby creating relative load data. The time-series load data associated with each section of the content is saved in the data saving unit 5 (Fig. 1) as relative load data corresponding to the content.

At this time, the data control unit 21 may add the relative load data to the content as metadata, or may associated it with the content as data different from the content.

Furthermore, the data control unit 21 edits the relative load data saved in the data saving unit 5 (Fig. 1) according to the operation information from the operation unit 24. The edited relative load data is saved in the data saving unit 5 again. The data control unit 21 may edit the relative load data input by the creator who is an operator operating the relative load creation/editing device 2.

The display control unit 22 controls the display unit 23 to display, on the display unit 23, a screen including the content and the UI for associating the time-series load data with each section of the content.

The display unit 23 includes a liquid crystal display, an organic electroluminescence (EL) display, or the like, and displays various kinds of information under the control of the display control unit 22.

The operation unit 24 includes a touch panel integrated with the display included in the display unit 23, a physical button provided on the housing of the relative load creation/editing device 2, and the like. The UI described above is displayed on the display unit 23 as a touch panel (operation unit 24). The operation information indicating the operation contents for the UI is supplied to the data control unit 21.

The data storage unit 25 stores programs required to operate the relative load creation/editing device 2 and various data prepared by the creator in advance. The data storage unit 25 also stores the time-series load data associated with each section of the content.

### (Operation of Relative Load Creation/Editing Device)

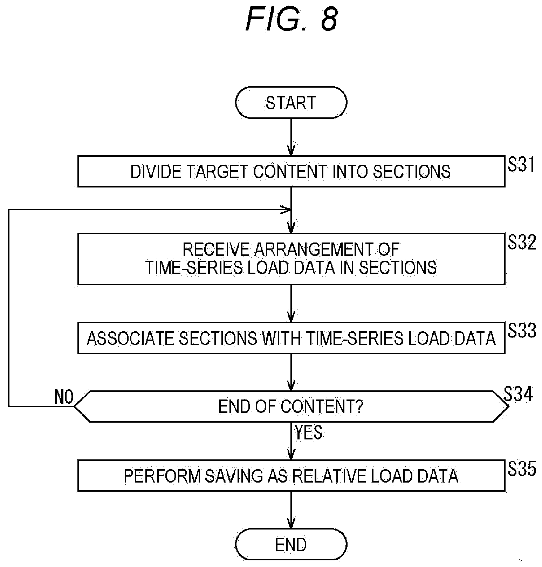

Next, a process of creating relative load data performed by the relative load creation/editing device 2 will be described with reference to a flowchart of Fig. 8.

In step S31, the data control unit 21 divides the target content to be associated with the relative load data into sections. The target content may be divided into sections divided at predetermined fixed time intervals such as 30 seconds, or may be divided into sections corresponding to contents of the content obtained by analyzing the contents.

In step S32, the operation unit 24 receives arrangement of the time-series load data in the sections.

In step S33, the data control unit 21 associates the sections with the time-series load data.

In step S34, the data control unit 21 determines whether or not the section associated with the time-series load data is the end of the content.

In a case where the section associated with the time-series load data is not determined to be the end of the content, the process returns to step S32, and the processing of steps S32 and S33 is repeated.

On the other hand, in a case where the section associated with the time-series load data is determined to be the end of the content, the process proceeds to step S35.

In step S35, the data control unit 21 saves, as relative load data, the time-series load data associated with each section of the content in the data saving unit 5.

The relative load data is created as described above.

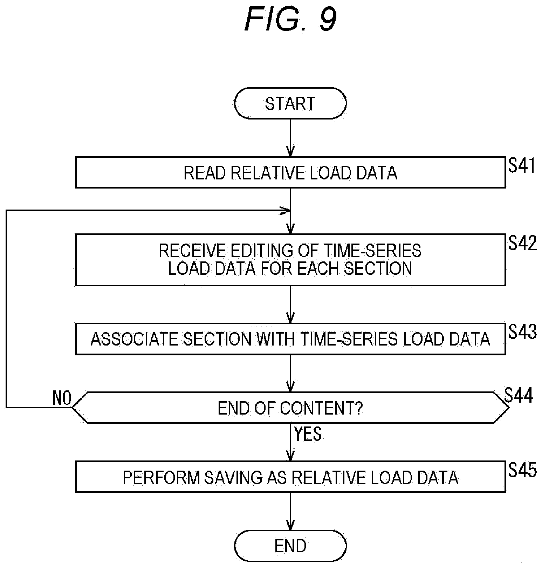

Next, a process of editing the relative load data performed by the relative load creation/editing device 2 will be described with reference to a flowchart of Fig. 9.

In step S41, the data control unit 21 reads the relative load data from the data saving unit 5. The relative load data read from the data saving unit 5 is, for example, relative load data associated with predetermined content in advance.

In step S42, the operation unit 24 receives editing of the time-series load data for each section. Specifically, the operation unit 24 receives a change in the magnitude of the relative load value of the time-series load data associated with the section selected by the user or a change in the order of the time-series load data. Then, the data control unit 21 changes the magnitude of the relative load value of the time-series load data, or changes the order of the time-series load data.

In step S43, the data control unit 21 associates the sections with the time-series load data.

In step S44, the data control unit 21 determines whether or not the section associated with the time-series load data is the end of the content.

In a case where the section associated with the time-series load data is not determined to be the end of the content, the process returns to step S42, and the processing of steps S42 and S43 is repeated.

On the other hand, in a case where the section associated with the time-series load data is determined to be the end of the content, the process proceeds to step S45.

In step S45, the data control unit 21 saves, as relative load data, the time-series load data associated with each section of the content in the data saving unit 5.

The relative load data is edited as described above.

### (Exemplary Screen Display)

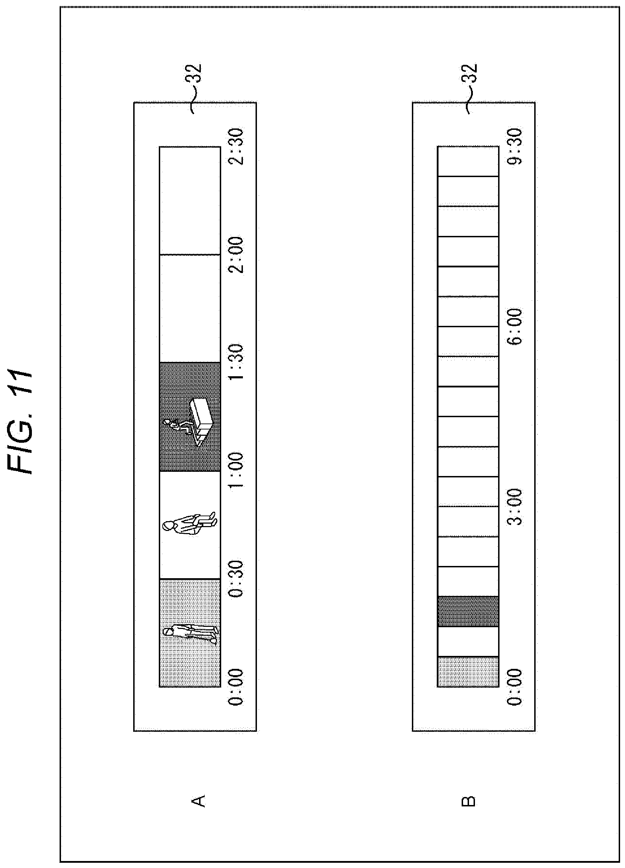

Fig. 11 is a diagram illustrating exemplary screen display for creating/editing the relative load data displayed on the display unit 23.

A screen illustrated in Fig. 11 includes four panels as display areas, which are a content panel 31, a section timeline panel 32, a time-series load timeline panel 33, and a load icon display panel 34. Note that the layout of each panel is not limited the exemplary screen illustrated in Fig. 11.

The content panel 31 is a panel on which video content to be associated with the time-series load data is displayed.

The section timeline panel 32 is a panel on which areas corresponding to respective sections obtained by temporally dividing the content displayed on the content panel 31 are displayed side by side. In the example of Fig. 11, areas corresponding to the sections for every 30 seconds from 0 min. 00 sec. to 2 min. 30 sec. of the content are displayed side by side in a horizontal line, and each area slides to the left in the section timeline panel 32 as time passes. Each section is associated with the time-series load data.

Furthermore, a reproduction point 35 indicating a temporal position currently being reproduced in the content displayed on the content panel 31 is displayed on the section timeline panel 32.

The time-series load timeline panel 33 is a panel that graphically displays temporal changes in the relative load value of the time-series load data associated with the section including the reproduction point 35 on the section timeline panel 32.

The load icon display panel 34 is an area in which a plurality of load icons 36 is displayed. In the example of Fig. 11, nine load icons 36 are displayed on the load icon display panel 34 in a 3 × 3 matrix.

Each of the load icons 36 is associated with a plurality of different types of time-series load data defined in advance. The load icon 36 is displayed in a color corresponding to the size of the associated time-series load data, and is displayed as an image corresponding to the size of the time-series load data. Specifically, the load icon 36 is displayed in a color representing the exercise intensity with an image of a person representing the exercise corresponding to the associated time-series load data.

For example, the upper three load icons 36 on the load icon display panel 34 depict images of persons performing daily actions such as sleeping, sitting, and standing, which are displayed in white. The middle three load icons 36 on the load icon display panel 34 depict images of persons performing light exercise such as cleaning, shopping, and dancing, which are displayed in light gray. The lower three load icons 36 on the load icon display panel 34 depict images of persons performing strenuous exercise such as jogging, dashing upstairs, and running, which are displayed in dark gray.

With the load icon 36 dragged and dropped from the load icon display panel 34 to an area corresponding to the section in the section timeline panel 32, the time-series load data is associated with the section corresponding to the area. At this time, in the section timeline panel 32, the load icon 36 is arranged in the area where the load icon 36 is dragged and dropped. Note that the operation of associating the time-series load data with the section corresponding to the area in the section timeline panel 32 may be an operation other than drag and drop, such as tapping (clicking) the area corresponding to the section and then tapping (clicking) the load icon 36, for example.

For example, when the lower center load icon 36 on the load icon display panel 34 is selected by a tap operation, the time-series load timeline panel 33 graphically displays the relative load value of the time-series load data associated with the selected load icon 36.

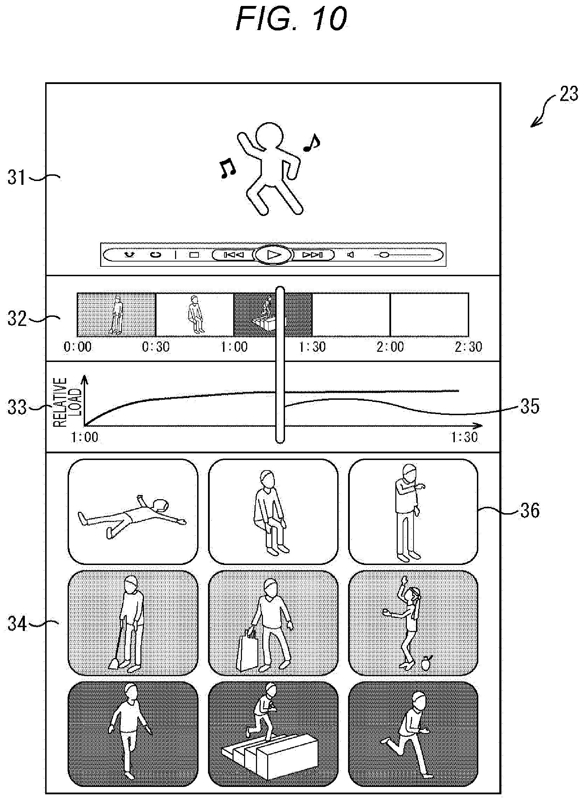

In the example of Fig. 10, the relative load value of the time-series load data in which the relative load value gradually increases from 0 (%VO2Max) to a certain value is graphically displayed.

When this load icon 36 is placed in the section from 1 min. 00 sec. to 1 min. 30 sec. on the section timeline panel 32, the time-series load data graphically displayed on the load icon display panel 34 is associated with the time period in the content displayed on the content panel 31.

According to the configuration described above, the creator who operates the relative load creation/editing device 2 is enabled to associate the time-series load data with each section of the content only by arranging the load data abstracted by the load icon in the section obtained by temporally diving the content. With this arrangement, it becomes possible to facilitate creation/editing of load data for providing a more realistic experience.

### (Display of Section Timeline Panel)

As described above, while the load icon 36 dragged and dropped from the load icon display panel 34 is displayed in each area of the section timeline panel 32, the number of the load icons 36 to be displayed may be changed.

For example, A of Fig. 11 illustrates the section timeline panel 32 in which five areas corresponding to the sections for every 30 seconds from 0 min. 00 sec. to 2 min. 30 sec. of the content are displayed side by side in a similar manner to the exemplary screen display of Fig. 10. In A in the drawing, an image of a person is drawn in the load icon arranged in each area, and is displayed in a predetermined color.

Meanwhile, B of Fig. 11 illustrates the section timeline panel 32 in which 18 areas corresponding to the sections for every 30 seconds from 0 min. 00 sec. to 9 min. 00 sec. of the content are displayed side by side. In B in the drawing, an image of a person is not drawn in the load icon arranged in each area, and is displayed only in a predetermined color.

Switching of the display of the section timeline panel 32 as illustrated in Fig. 11 is performed by, for example, an operation of a slider (not illustrated) for zooming in and out or a pinch-in/pinch-out operation on the section timeline panel 32.

### (Relative Load Value at Section Boundary)

In the graph displayed on the time-series load timeline panel 33, there is a case where the relative load value at a boundary of sections, that is, the first relative load value of the current section is different from the last relative load value of the previous section.

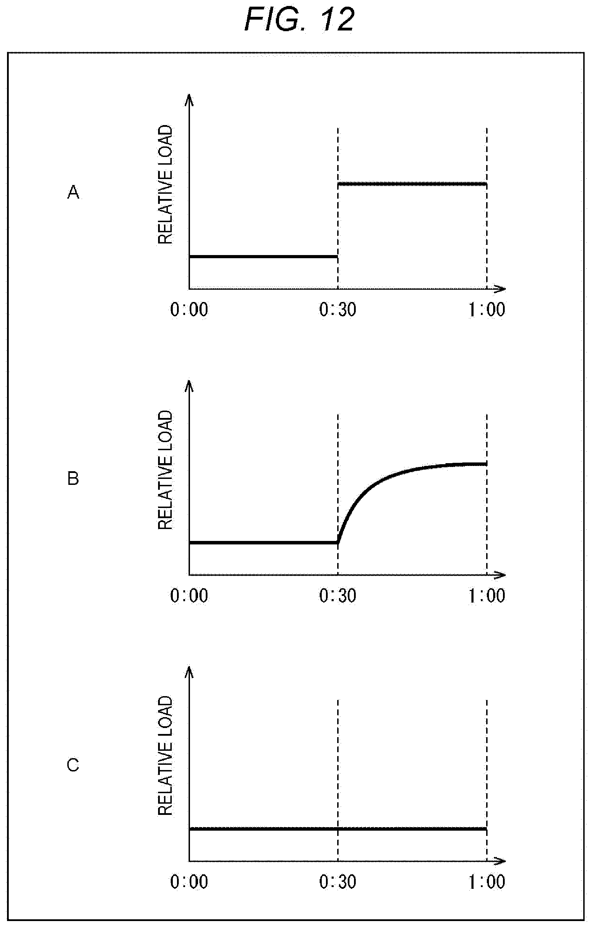

Fig. 12 is a diagram illustrating an exemplary graph of the time-series load data (relative load data) in a case where the first relative load value of the current section is different from the last relative load value of the previous section.

Each of A, B, and C of Fig. 12 illustrates the time-series load data for two sections from 0 min. 00 sec. to 1 min. 00 sec. of the content. In the example of Fig. 12, the last relative load value of the section from 0 min. 00 sec. to 0 min. 30 sec. and the first relative load value of the section from 0 min. 30 sec. to 1 min. 00 sec. take different values.

In such a case, as illustrated in A in the drawing, the relative load data may be created as it is without matching the last relative load value of the section from 0 min. 00 sec. to 0 min. 30 sec. and the first relative load value of the section from 0 min. 30 sec. to 1 min. 00 sec.

Furthermore, as illustrated in B in the drawing, the relative load value of the section from 0 min. 30 sec. to 1 min. 00 sec. may be smoothed from the last relative load value of the section from 0 min. 00 sec. to 0 min. 30 sec. to create the relative load data.

Moreover, as illustrated in C in the drawing, the offset of the first relative load value of the section from 0 min. 30 sec. to 1 min. 00 sec. may be matched with the last relative load value of the section from 0 min. 00 sec. to 0 min. 30 sec. to create the relative load data.

### (Display of Content Panel)

While the video content is displayed on the content panel 31 in the example of Fig. 10, items other than the video content may be displayed.

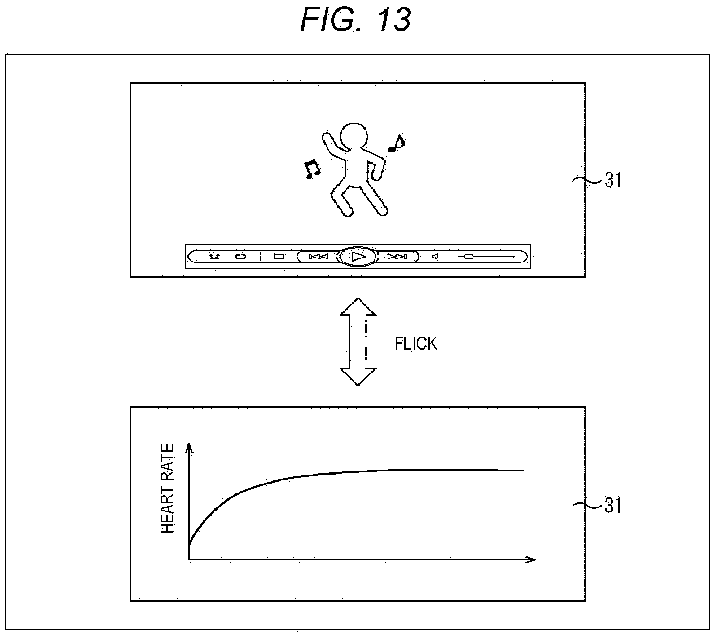

For example, as illustrated in Fig. 13, when a flick operation is performed on the content panel 31, video content and a graph of the heart rate that the creator wants to refer to are switched and displayed on the content panel 31.

Furthermore, for example, when a normalization parameter of an arbitrary user A is obtained, it is possible to convert the created relative load data into an actual load (absolute load data) for the user A. In such a case, a simulation moving image in which an avatar of the user A is running at a speed corresponding to the converted load may be displayed on the content panel 31. The creator is enabled to adjust the relative load data while viewing the simulation moving image displayed on the content panel 31, and to sell a simulation moving image (avatar) corresponding to the adjusted relative load data.

### (Display of Load Icon Display Panel)

While nine load icons 36 are displayed on the load icon display panel 34 in the example of Fig. 11, a larger number of the load icons 36 may be displayed by being scrollable up, down, right, and left.

Furthermore, the load icons 36 displayed on the load icon display panel 34 may have a hierarchical structure.

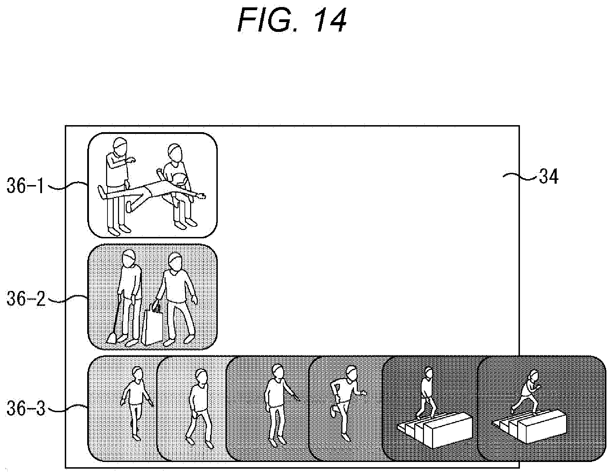

For example, as illustrated in Fig. 14, load icons 36-1, 36-2, and 36-3 in which the same type of exercise is combined into one, such as for each exercise intensity, may be displayed on the load icon display panel 34.

Then, individual load icons corresponding to the exercise put together by the load icon selected by a tap operation or the like may be expanded in one direction. In the example of Fig. 14, six load icons corresponding to the exercise put together by the load icon 36-3 are expanded in the right direction.

Moreover, the arrangement of the load icons 36 may be customized such that the load icons 36 displayed on the load icon display panel 34 are arranged in a sorted manner according to the magnitude of the relative load value of the associated time-series load data or the like.

Furthermore, among the plurality of load icons 36, the load icon 36 corresponding to the contents of the video content displayed on the content panel 31 may be displayed in a prioritized manner in the load icon display panel 34. Specifically, the action in the video content displayed on the content panel 31 is recognized, and the load icon 36 corresponding to the recognition result is displayed on the load icon display panel 34. For example, in a case where the action of dashing upstairs is recognized in the video content, the load icon 36 corresponding to the action of dashing upstairs is displayed on the load icon display panel 34 in a prioritized manner. With this arrangement, it becomes possible to easily search for the load icon 36 corresponding to the video content displayed on the content panel 31.

Furthermore, while the video content is displayed on the content panel 31, the load icon recommended for each section (video content in the time period) may be displayed (recommendation display) in the area corresponding to the section in the section timeline panel 32, for example. With this arrangement, it becomes possible to more easily create/edit the relative load data matching the video content.

### (Time-Series Load Data Editing)

The relative load value of the time-series load data graphically displayed on the time-series load timeline panel 33 is one-dimensional time-series data, which can be edited by an operation made on the time-series load timeline panel 33.

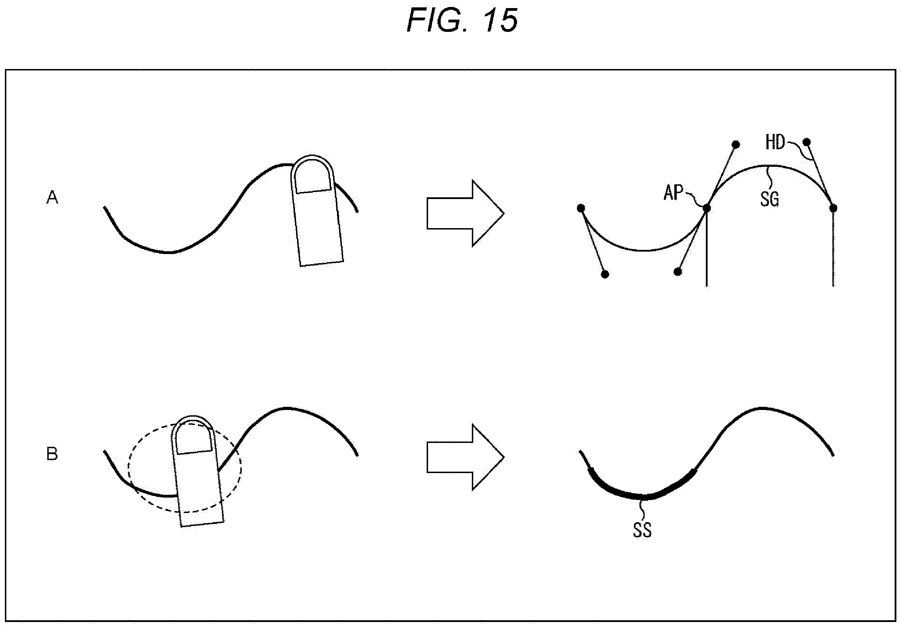

For example, when a graph of the time-series load data is selected by a tap operation with a finger as illustrated on the left side of A of Fig. 15, an anchor point AP and a handle HD are displayed on the graph as illustrated on the right side of A in the drawing.

The anchor point AP is indicated by a value (point) inevitably taken by the graph, and the graph is drawn by a segment SG connecting the anchor points AP.

The handle HD is indicated by a straight line drawn from the anchor point AP, and a curvature of the segment SG is adjusted by an inclination of the handle HD.

In the example of A in the drawing, with the anchor point AP and the handle HD dragged and dropped, the graph of the time-series load data is made editable in units of points.

Furthermore, when an area including the graph of the time-series load data (area surrounded by a broken line in the drawing) is selected with a finger as illustrated on the left side of B of Fig. 15, a selected section SS in the graph is emphasized and displayed as illustrated on the right side of B in the drawing.

In the example of B in the drawing, with the sections SS dragged and dropped, the sections SS in the graph of the time-series load data can be collectively subject to offset adjustment.

### (Load Icon Editing)

When the graph of the time-series load data is edited on the time-series load timeline panel 33, display of the load icon 36 arranged in the corresponding section on the section timeline panel 32 may change. For example, with a star sign added to the load icon 36 arranged in the corresponding section on the section timeline panel 32, it is clearly indicated that the corresponding graph of the time-series load data has been edited.

Furthermore, the load icon 36 in which the graph of the time-series load data has been edited may be saved as a new load icon 36 by, for example, the corresponding section of the section timeline panel 32 subject to a long-press or the like. At this time, the color of the load icon 36 may be automatically set, or may be selected by the user. Furthermore, an image of a person to be drawn on the load icon 36 may be optionally edited by the user.

Moreover, the load icons 36 arranged in a plurality of sections on the section timeline panel 32 may be saved as a combined load icon (hereinafter referred to as recipe load icon) by, for example, the corresponding plurality of sections on the section timeline panel 32 subject to a long-press or the like. In this case, the time-series load data associated with the plurality of load icons 36 put together by the recipe load icon is also edited to be combined into one piece of time-series load data.

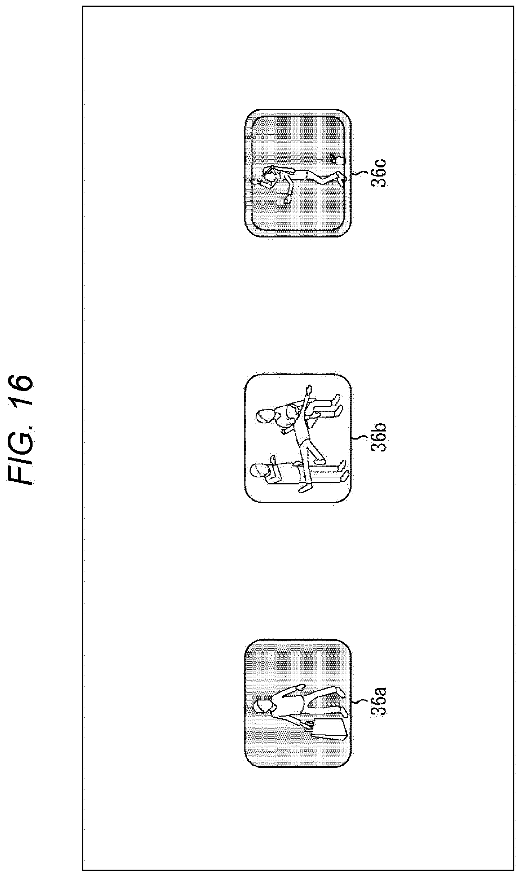

Fig. 16 is a diagram illustrating exemplary display of the load icon 36 displayed on the load icon display panel 34.

In Fig. 16, a load icon 36a is an exemplary normal load icon. In the load icon 36a, only one image of a person performing exercise corresponding to the corresponding time-series data is drawn in a frame.

A load icon 36b is an exemplary load icon having the hierarchical structure described with reference to Fig. 14. In the load icon 36b, for example, an image of a plurality of persons performing exercise corresponding to the time-series load data hierarchized by exercise intensity or the like is drawn in a frame.

A load icon 36c is an exemplary recipe load icon. In the load icon 36c, frames are doubly displayed, and for example, images of persons drawn in a plurality of load icons 36 combined into one are displayed as a moving image.

As described above, the newly saved load icon or recipe load icon may be shared with another person or traded via a network such as the Internet.

Furthermore, in the relative load calculation device 1, the load icon corresponding to the time-series data obtained by dividing the relative load data obtained on the basis of the actual exercise of the recorded person may be newly saved or shared in a similar manner to the load icons described above.

### (Load Icon Personalization by Creator)

As described above, the time-series load data is abstracted by the image or color of the load icon 36 and is displayed. However, depending on the creator, the image and color of the load icon 36 may not be suited for the sense of the individual person.

For example, it is assumed that an icon creator who has significantly high VO2Max, that is, who is very good at exercising sets a load icon representing "walking at 6 km/h" for time-series load data corresponding to exercise of maintaining 20% VO2Max for a long period of time.

However, a relative load of "walking at 6 km/h" is high (e.g., corresponding to 40% VO2Max) for a user (creator) who has significantly low VO2Max, and the load icon does not match the sense of the creator.

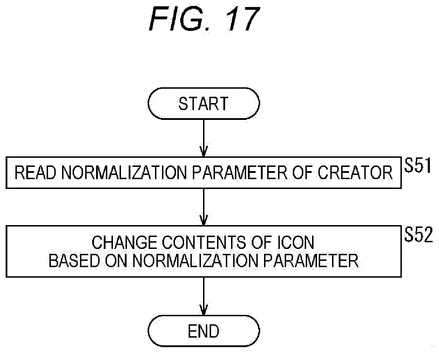

In view of the above, a process of changing display (load icon personalization) for changing display of the icon on the basis of the normalization parameter of the creator will be described with reference to a flowchart of Fig. 17.

In step S51, the data control unit 21 reads the normalization parameter of the creator.

In step S52, the data control unit 21 changes the contents of the icon corresponding to the target time-series load data on the basis of the read normalization parameter of the creator. For example, in the example described above, the load icon representing "walking at 6 km/h" is changed to a load icon representing "jogging at 8 km/h".

With this arrangement, it becomes possible to match the load icon with the sense of the creator.

Note that the process of Fig. 17 may be executed by the load icon to be changed selected by the creator, or may be collectively executed for a plurality of load icons according to a table prepared in advance.

### <4. Relative Load Data Reproduction>

Hereinafter, a configuration of the relative load reproduction device 3 that provides the player with load experience on the basis of the relative load data calculated by the relative load calculation device 1 or created/edited by the relative load creation/editing device 2 will be described.

### (Configuration of Relative Load Reproduction Device)

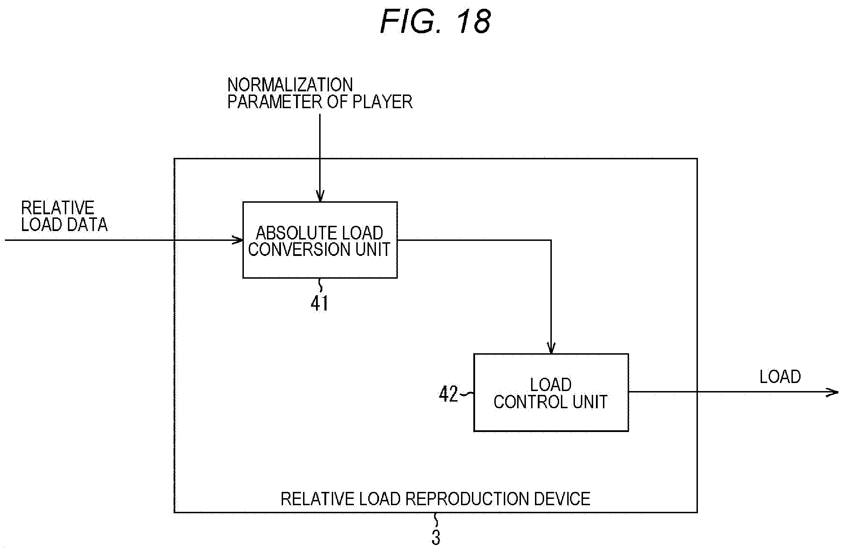

Fig. 18 is a block diagram illustrating an exemplary configuration of the relative load reproduction device 3.

The relative load reproduction device 3 of Fig. 18 includes an absolute load conversion unit 41 and a load control unit 42.

The absolute load conversion unit 41 converts the relative load data saved in the data saving unit 5 into absolute load data on the basis of the normalization parameter of the player, and supplies it to the load control unit 42.

The load control unit 42 controls the load provided to the player in a load providing device worn or used by the player on the basis of the absolute load data from the absolute load conversion unit 41.

### (Operation of Relative Load Reproduction Device)

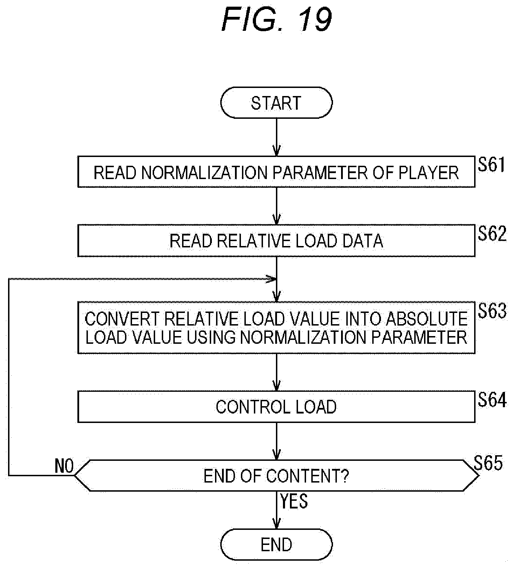

Next, a process of reproducing the relative load data performed by the relative load reproduction device 3 will be described with reference to a flowchart of Fig. 19. The process of Fig. 19 starts when the content associated with the relative load data is reproduced.

In step S61, the absolute load conversion unit 41 reads the normalization parameter of the player.

In step S62, the absolute load conversion unit 41 reads the relative load data saved in the data saving unit 5.

In step S63, the absolute load conversion unit 41 converts the relative load value of the relative load data into an absolute load value using the normalization parameter of the player.

In step S64, the load control unit 42 controls the load provided from the load providing device on the basis of the converted absolute load value.

In step S65, the absolute load conversion unit 41 determines whether or not the content being reproduced is the end of the content.

In a case where it is determined not to be the end of the content, the process returns to step S63, and the processing of steps S63 and S64 is repeated.

On the other hand, in a case where it is determined to be the end of the content, the process is terminated.

In this manner, the player is enabled to experience the load according to his/her physical ability.

### (Another Configuration of Relative Load Reproduction Device)

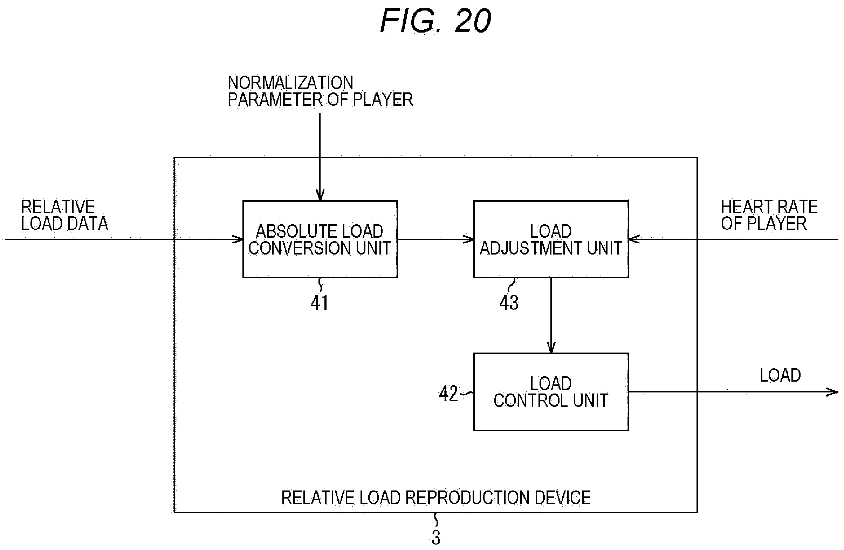

Fig. 20 is a block diagram illustrating another exemplary configuration of the relative load reproduction device 3.

The relative load reproduction device 3 of Fig. 20 further includes a load adjustment unit 43 in addition to the configuration similar to the relative load reproduction device 3 of Fig. 18.

The load adjustment unit 43 obtains a heart rate of the player, and compares the load data based on the heart rate with the absolute load data from the absolute load conversion unit 41, thereby adjusting the absolute load data and eventually the load provided to the player in the load providing device.

The load control unit 42 controls the load provided in the load providing device on the basis of the absolute load data adjusted by the load adjustment unit 43.

### (Operation of Relative Load Reproduction Device)

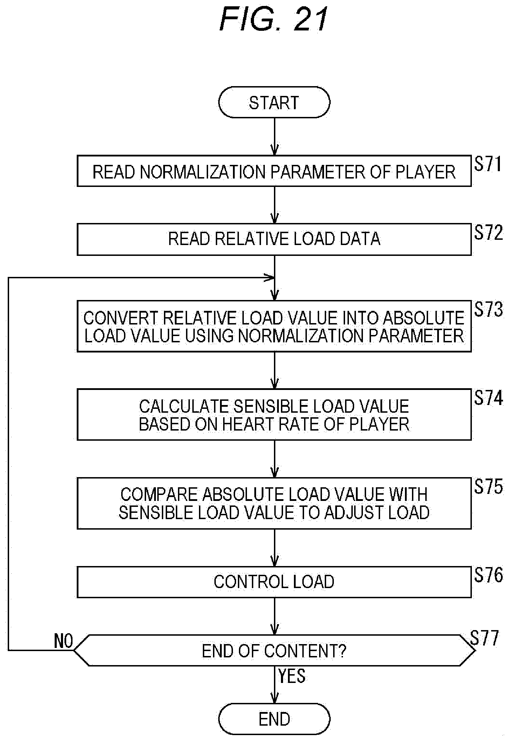

Next, a process of reproducing the relative load data performed by the relative load reproduction device 3 of Fig. 20 will be described with reference to a flowchart of Fig. 21.

Note that the processing of steps S71 to S73 in the flowchart of Fig. 21 is similar to the processing of steps S61 to S63 in the flowchart of Fig. 19, and thus the descriptions thereof will be omitted.

That is, after the relative load value of the relative load data is converted into the absolute load value using the normalization parameter of the player, in step S74, the load adjustment unit 43 calculates a sensible load value on the basis of the heart rate of the player.

In step S75, the load adjustment unit 43 compares the absolute load value with the sensible load value, thereby adjusting the absolute load value.

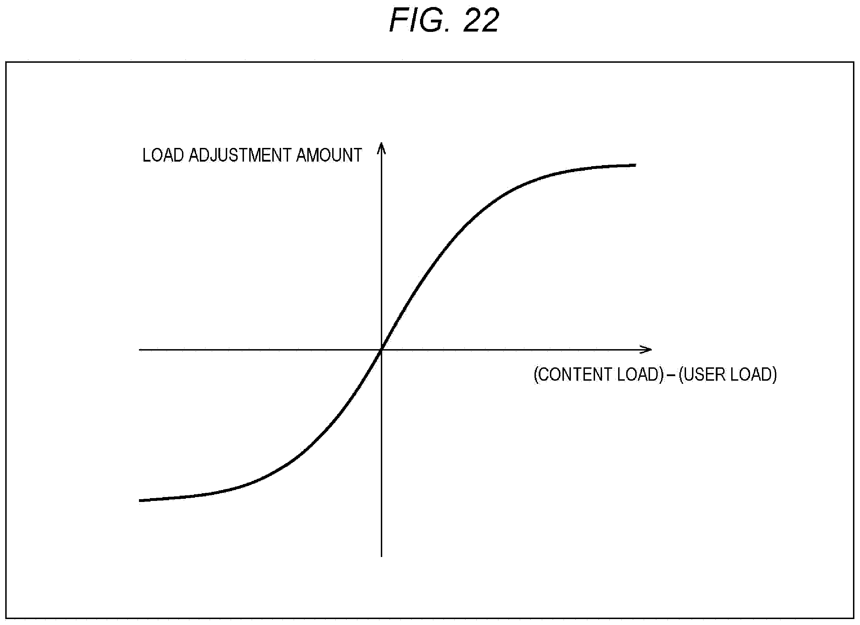

For example, as illustrated in Fig. 22, in a case where the user load indicated by the sensible load value is larger than the content load indicated by the absolute load value (in a case where (content load) - (user load) > 0), the absolute load value is adjusted in such a manner that the load provided in the load providing device becomes larger.

On the other hand, in a case where the user load indicated by the sensible load value is smaller than the content load indicated by the absolute load value (in a case where (content load) - (user load) < 0), the absolute load value is adjusted in such a manner that the load provided in the load providing device becomes smaller.

When the absolute load value is adjusted in this manner, in step S76, the load control unit 42 controls the load provided from the load providing device on the basis of the adjusted absolute load value.

In step S77, the absolute load conversion unit 41 determines whether or not the content being reproduced is the end of the content.

In a case where it is determined not to be the end of the content, the process returns to step S73, and the processing of steps S73 to S76 is repeated.

On the other hand, in a case where it is determined to be the end of the content, the process is terminated.

In this manner, the player is enabled to experience the load according to his/her physical ability in consideration of his/her physical condition at that time.

### (Exemplary Load Providing Device)

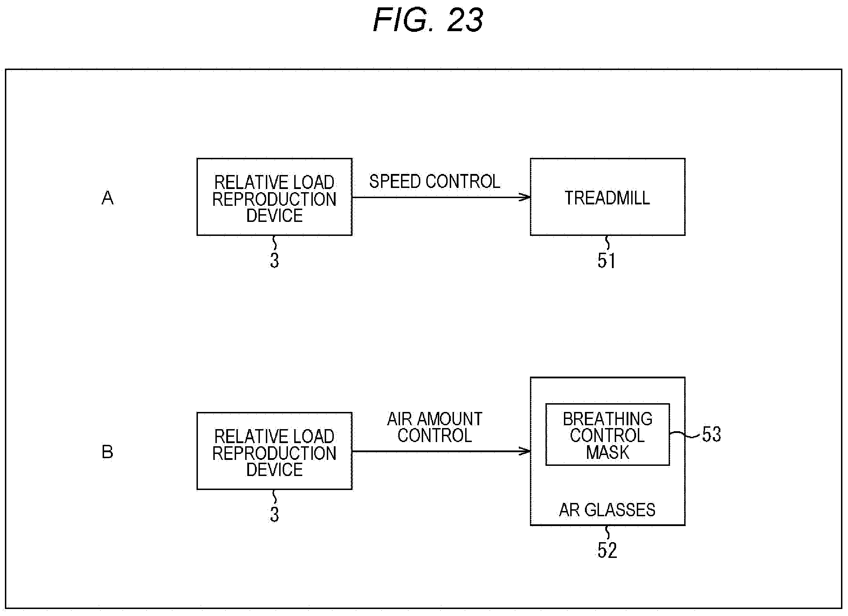

Fig. 23 is a diagram illustrating an exemplary load providing device to be controlled by the relative load reproduction device 3.

A of Fig. 23 illustrates an example in which the relative load reproduction device 3 controls a treadmill 51 as a load providing device. In A in the drawing, the relative load reproduction device 3 controls a speed of the treadmill 51, thereby controlling the load provided to the player.

B of Fig. 23 illustrates an example in which the relative load reproduction device 3 controls augmented reality (AR) glasses 52 as a load providing device. The AR glasses 52 are configured integrally with a breathing control mask 53, and present content to the player. The breathing control mask 53 adjusts a degree of an air intake by opening/closing a valve, thereby controlling the ease of breathing of the player wearing the AR glasses 52. That is, in B in the drawing, the relative load reproduction device 3 controls the air amount of the breathing control mask 53, thereby controlling the load provided to the player. With this arrangement, the player is enabled to experience the load that increases the heart rate by entering hypoxic conditions by the breathing control mask 53.

Note that, in B of Fig. 23, the relative load reproduction device 3 may control virtual reality (VR) glasses instead of the AR glasses 52.

### (Application of Relative Load Reproduction Device)

In recent years, online lessons for carrying out training or yoga while watching moving images distributed to a PC or a smartphone have become widespread. However, in such an online lesson, it is not possible to know whether or not the user is exercising according to a menu.

Even if a motion of the user can be grasped by a motion capture or the like, it is not possible to know whether or not respiration is performed with a correct rhythm in yoga or the like.

Meanwhile, according to the relative load reproduction device 3 of B of Fig. 23, it becomes possible to distribute a correct breathing rhythm in addition to moving images. Specifically, with the relative load reproduction device 3 controlling the air amount of the breathing control mask 53 with a predetermined rhythm, the user is enabled to exercise with the correct breathing rhythm.

The breathing rhythm may be calculated from the breathing rhythm of the recorded person using the relative load calculation device 1 as the relative load data described above, or may be created/edited by the creator using the relative load creation/editing device 2.

Furthermore, in yoga or the like, a display or a sound for calling attention to the user may be output in a case where the breathing rhythm of the user is obtained from the blood pulse wave and a difference from the distributed breathing rhythm is detected to have increased by the user stopping breathing or the like.

### <5. Normalization Parameter Acquisition>

In a case where the content associated with the relative load data as described above has been widely known, it is preferable that the normalization parameter of the user (player) can be easily obtained.

While a method of obtaining the normalization parameter of the user has been conventionally known, this method is suitable for athletes, and requires high-load exercise in advance.

In view of the above, the normalization parameter calculation device 4 calculates a normalization parameter of the player on the basis of the daily log including exercise data and biological information in daily life of the player, thereby making it possible to obtain the normalization parameter from low-load exercise.

### (Configuration of Normalization Parameter Calculation Device)

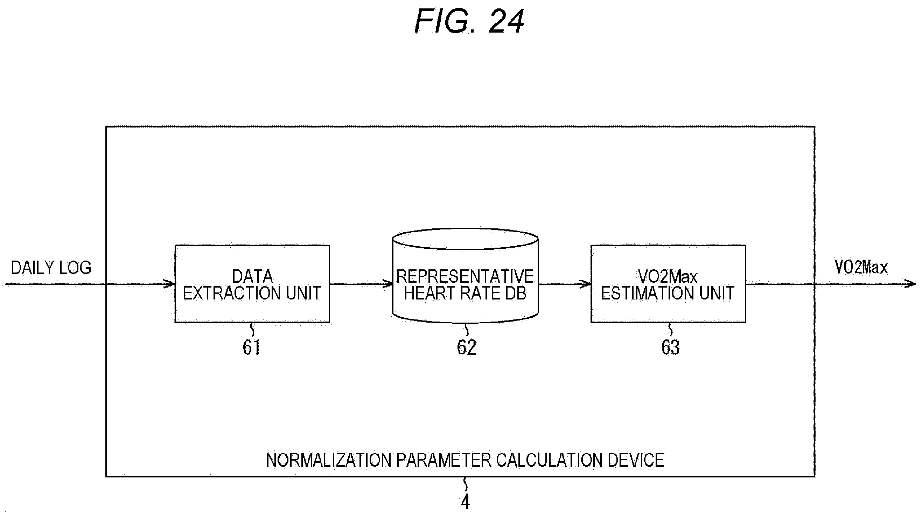

Fig. 24 is a block diagram illustrating an exemplary configuration of the normalization parameter calculation device 4.

The normalization parameter calculation device 4 of Fig. 24 includes a data extraction unit 61, a representative heart rate DB 62, and a VO2Max estimation unit 63.

The data extraction unit 61 extracts a speed-specific representative heart rate, which is a representative value of the heart rate for each walking speed, on the basis of the daily log of the player, and supplies it to the representative heart rate DB 62.

The representative heart rate DB 62 stores the speed-specific representative heart rate from the data extraction unit 61. The stored speed-specific representative heart rate is read by the VO2Max estimation unit 63 as appropriate.

The VO2Max estimation unit 63 estimates the VO2Max of the player as a normalization parameter of the player on the basis of the speed-specific representative heart rate stored in the representative heart rate DB 62.

### (Configuration and Operation of Data Extraction Unit)

Here, a configuration and operation of the data extraction unit 61 will be described.

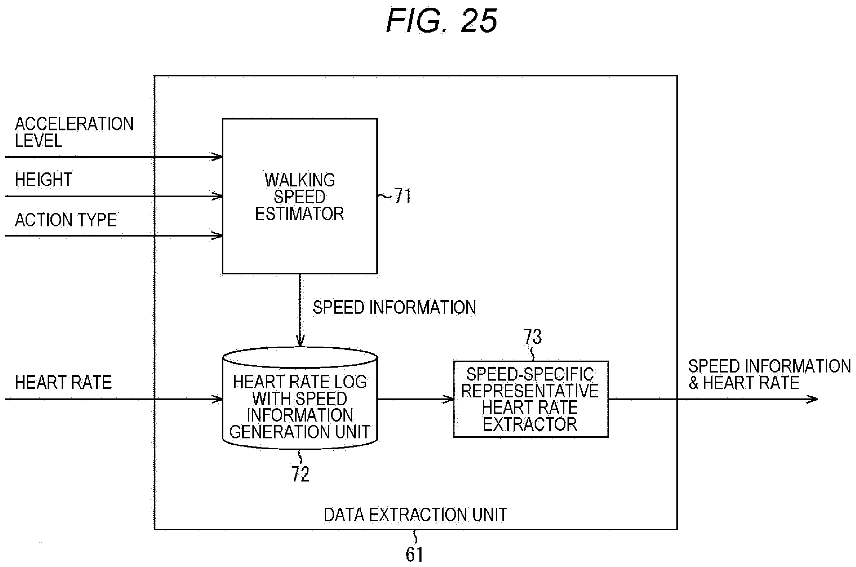

Fig. 25 is a block diagram illustrating an exemplary configuration of the data extraction unit 61.

The data extraction unit 61 includes a walking speed estimator 71, a heart rate log with speed information generation unit 72, and a speed-specific representative heart rate extractor 73.

The walking speed estimator 71 estimates a time-series walking speed of the player on the basis of the acceleration level during walking of the player, the height and the action type of the player, which are obtained as a daily log. The action type indicates a type of the action of the player, which may be obtained as a result of the action recognition of the player, or may be obtained by being set by the player. The speed information indicating the estimated walking speed is supplied to the heart rate log with speed information generation unit 72.

The heart rate log with speed information generation unit 72 merges the speed information from the walking speed estimator 71 with the heart rate at the time of walking of the player obtained as a daily log, thereby generating a heart rate log with speed information, which is time-series data.

The speed-specific representative heart rate extractor 73 extracts, for each speed information, the heart rate in a stable state in the heart rate log with speed information generated by the heart rate log with speed information generation unit 72, and saves it in the representative heart rate DB 62 as a speed-specific representative heart rate.

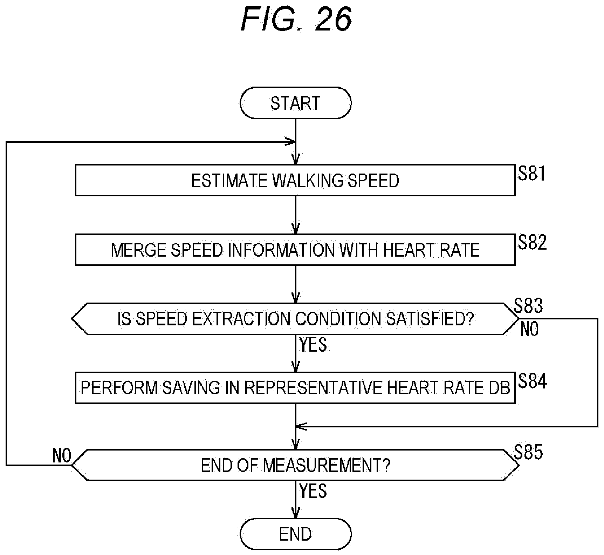

Fig. 26 is a flowchart for explaining operation of the data extraction unit 61. The process of Fig. 26 starts together with measurement of a daily log of the player.

In step S81, the walking speed estimator 71 estimates a time-series walking speed of the player on the basis of the daily log.

In step S82, the heart rate log with speed information generation unit 72 merges the speed information indicating the walking speed estimated by the walking speed estimator 71 with the heart rate of the player, thereby generating a heart rate log with speed information.

In step S83, the speed-specific representative heart rate extractor 73 determines whether or not the heart rate satisfies a speed extraction condition in the heart rate log with speed information.

In general, the heart rate immediately after the start of exercise shows a rapidly increasing transient response, and thus it is not suitable for use as a representative value. In view of the above, in step S83, it is determined whether or not the heart rate satisfies the speed extraction condition depending on whether or not the heart rate is in a stable state.

In a case where the heart rate is determined to satisfy the speed extraction condition, that is, in a case where the heart rate is determined to be in a stable state, the process proceeds to step S84.

In step S84, the speed-specific representative heart rate extractor 73 extracts the heart rate in the stable state as a speed-specific representative heart rate corresponding to the speed information, and saves it in the representative heart rate DB 62.

On the other hand, in a case where the heart rate is determined not to satisfy the speed extraction condition, that is, in a case where the heart rate is determined not to be in a stable state in step S83, step S84 is skipped.

In step S85, the data extraction unit 61 determines whether or not the measurement of the daily log is complete.

In a case where the measurement of the daily log is determined not to be complete, the process returns to step S81, and the subsequent steps are repeated. On the other hand, in a case where the measurement of the daily log is determined to be complete, the process is terminated.

As described above, the speed-specific representative heart rate is extracted, and is saved in the representative heart rate DB 62. Note that, in a case where the speed-specific representative heart rates are not sufficiently saved in the representative heart rate DB 62, the normalization parameter calculation device 4 may output a message indicating that circumstances to prompt the player to measure the daily log.

### (Configuration and Operation of VO2Max Estimation Unit 63)

Next, a configuration and operation of the VO2Max estimation unit 63 will be described.

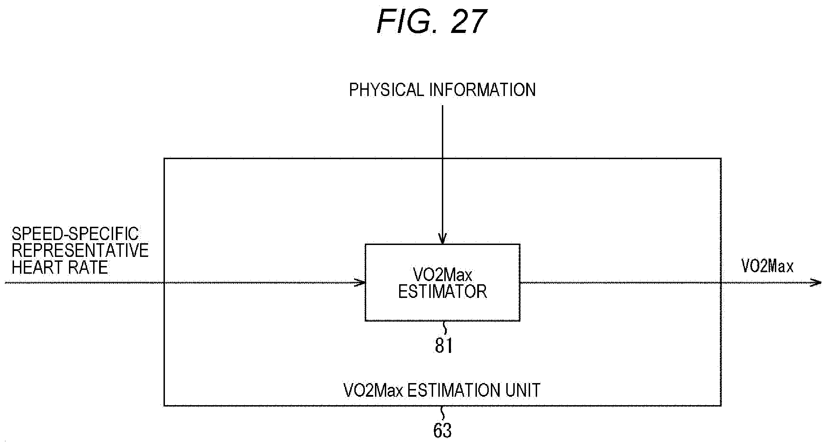

Fig. 27 is a block diagram illustrating an exemplary configuration of the VO2Max estimation unit 63.

The VO2Max estimation unit 63 includes a VO2Max estimator 81.

The VO2Max estimator 81 estimates VO2Max of the player using a plurality of speed-specific representative heart rates saved in the speed-specific representative heart rate extractor 73. At this time, VO2Max of the player may be estimated in consideration of physical information input by the player, specifically, the height, weight, age, gender, and the like.

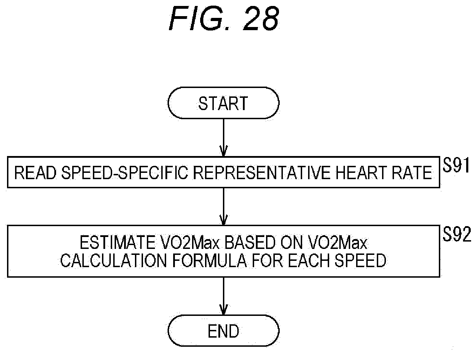

Fig. 28 is a flowchart for explaining operation of the VO2Max estimation unit 63. The process of Fig. 28 is executed at an optional timing.

In step S91, the VO2Max estimator 81 reads the speed-specific representative heart rate saved in the representative heart rate DB 62.

In step S92, the VO2Max estimator 81 estimates the VO2Max of the player using the speed-specific representative heart rate on the basis of a VO2Max calculation formula for each speed.

In general, the oxygen intake (VO2) and the exercise intensity have a proportional relationship until reaching the VO2Max. Meanwhile, the heart rate linearly increases as the exercise intensity increases. Therefore, the oxygen intake (VO2) and the heart rate have a proportional relationship.

Accordingly, with the VO2Max calculated for each exercise intensity, that is, for each speed, it becomes possible to estimate the ultimate VO2Max.

As described above, the VO2Max of the player is estimated.

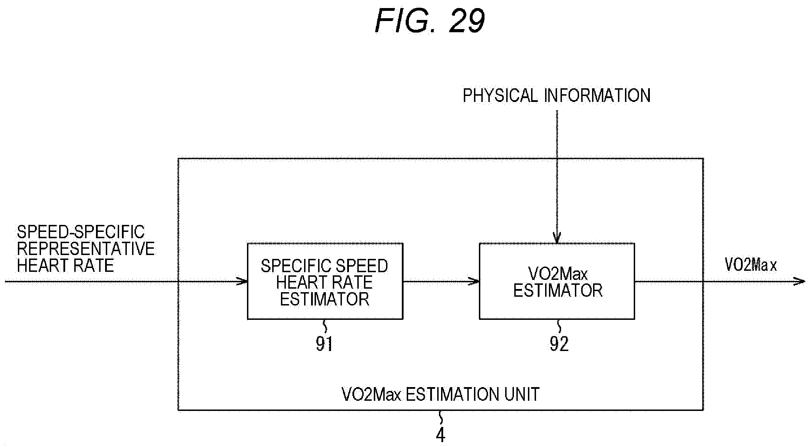

Fig. 29 is a block diagram illustrating another exemplary configuration of the VO2Max estimation unit 63.

The VO2Max estimation unit 63 of Fig. 29 includes a specific speed heart rate estimator 91 and a VO2Max estimator 92.

The specific speed heart rate estimator 91 estimates a heart rate at a specific speed on the basis of a plurality of the speed-specific representative heart rates saved in the speed-specific representative heart rate extractor 73.

The VO2Max estimator 92 estimates VO2Max of the player using a heart rate at a specific speed. At this time, VO2Max of the player may be estimated in consideration of physical information input by the player, specifically, the height, weight, age, gender, and the like.

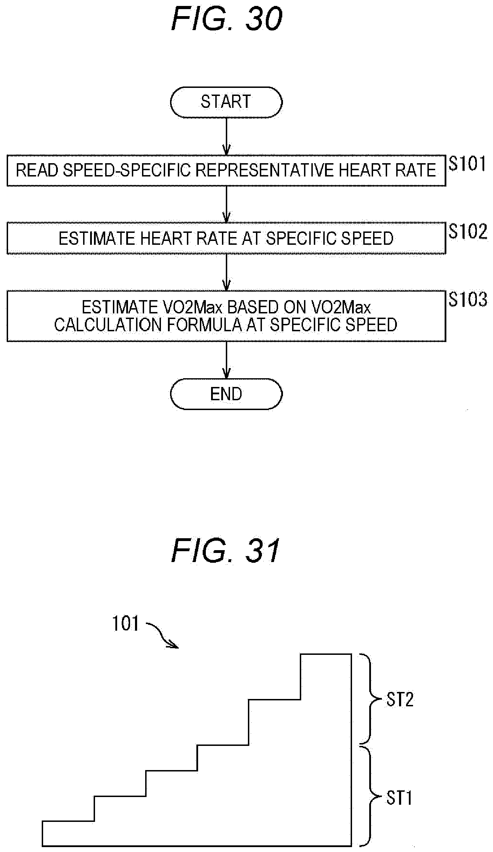

Fig. 30 is a flowchart for explaining the operation of the VO2Max estimation unit 63 of Fig. 29. The process of Fig. 30 is also executed at an optional timing.

In step S101, the specific speed heart rate estimator 91 reads a plurality of the speed-specific representative heart rates saved in the representative heart rate DB 62.

In step S102, the specific speed heart rate estimator 91 estimates a heart rate at a specific speed on the basis of the read plurality of speed-specific representative heart rates.

In step S103, the VO2Max estimator 92 estimates VO2Max of the player using the heart rate at the specific speed on the basis of a VO2Max calculation formula at the specific speed.

In this example, it is possible to estimate the VO2Max using only one VO2Max calculation formula.

As described above, the VO2Max of the player is estimated.

According to the configuration and processing described above, it becomes possible to obtain the VO2Max as the normalization parameter of the player from only the low-load daily log.

### (Normalization Parameter Acquisition before Event)

In a case of providing a load experience with realistic feeling in an event such as a movie or a concert, there may be an audience who has not obtained the above-described normalization parameter based on the daily log. It is necessary to cause such an audience to obtain the normalization parameter in a short period of time before the event.

In view of the above, in such a case, the normalization parameter calculation device 4 measures a heart rate with a known load to calculate the normalization parameter, in a similar manner to the daily log.

For example, a heart rate at the time of viewing an advertising announcement before a movie starts or at the time of performing a physical exercise for obtaining a normalization parameter (VO2Max) may be measured.

Furthermore, as illustrated in Fig. 31, it is also possible to place, for example, stairs 101 having different heights per step between a lower part ST1 of four steps and an upper part ST2 of two steps in a venue and to adjust a speed by a crowd, whereby VO2Max of the audience can be calculated.

For example, the oxygen intake in a stepping exercise can be obtained by the sum of the oxygen intake "0.35 × speed (cnt/min)" for work in the horizontal direction and the oxygen intake "step height (m) × speed (cnt/min) × 1.33 × 1.8" for work in the vertical direction.

The VO2Max of the audience may be calculated on the basis of such an expression.

As described above, in a case of providing a load experience in an event such as a movie or a concert, it becomes possible to cause the audience who has not obtained the normalization parameter to obtain the normalization parameter in a short period of time before the event.

### <6. Exemplary Computer Configuration>

The series of processing described above may be executed by hardware, or may be executed by software. In a case where the series of processing is executed by software, a program included in the software is installed in a computer. Here, examples of the computer include a computer incorporated in dedicated hardware, a general-purpose personal computer capable of implementing various functions by installing various programs, and the like.

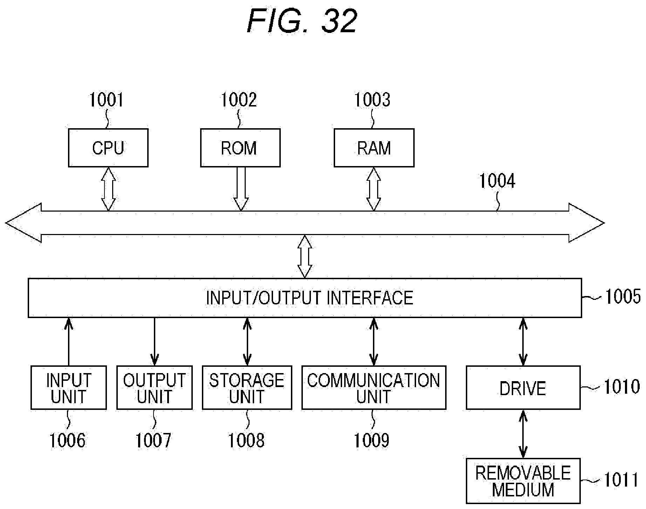

Fig. 32 is a block diagram illustrating an exemplary hardware configuration of a computer that executes, using a program, the series of processing described above.

In the computer, a central processing unit (CPU) 1001, a read only memory (ROM) 1002, and a random access memory (RAM) 1003 are coupled to one another via a bus 1004.

An input/output interface 1005 is further connected to the bus 1004. An input unit 1006, an output unit 1007, a storage unit 1008, a communication unit 1009, and a drive 1010 are connected to the input/output interface 1005.

The input unit 1006 includes a keyboard, a mouse, a microphone, and the like. The output unit 1007 includes a display, a speaker, and the like. The storage unit 1008 includes a hard disk, a non-volatile memory, and the like. The communication unit 1009 includes a network interface, and the like. The drive 1010 drives a removable medium 1011 such as a magnetic disk, an optical disk, a magneto-optical disk, or a semiconductor memory.

In the computer configured as described above, for example, the CPU 1001 loads the program stored in the storage unit 1008 into the RAM 1003 via the input/output interface 1005 and the bus 1004, and executes the program, thereby performing the series of processing described above.

The program to be executed by the computer (CPU 1001) may be provided by, for example, being recorded in the removable medium 1011 as a package medium or the like. Furthermore, the program may be provided through a wired or wireless transmission medium such as a local area network, the Internet, and digital satellite broadcasting.

In the computer, the program may be installed in the storage unit 1008 via the input/output interface 1005 by attaching the removable medium 1011 to the drive 1010. Furthermore, the program may be received by the communication unit 1009 via a wired or wireless transmission medium and installed in the storage unit 1008. In addition, the program may be installed in the ROM 1002 or the storage unit 1008 in advance.

Note that the program to be executed by the computer may be a program in which processing is executed in a time-series manner according to the order described herein, or may be a program in which processing is executed in parallel or at a necessary timing such as when a call is made.

Furthermore, in the present specification, steps for writing a program to be recorded in a recording medium are not only the processing executed in a time-series manner according to the described order, but also processing executed in parallel or individually without necessarily being processed in a time-series manner.

Moreover, the embodiment of the technology according to the present disclosure is not limited to the embodiment described above, and various modifications may be made without departing from the gist of the technology according to the present disclosure.

Furthermore, the effects described herein are merely examples and not limited, and additional effects may be included.

Moreover, the technology according to the present disclosure may employ the following configurations.
(1) An information processing apparatus including:
   a data control unit that associates relative load data, which is time-series load data relative to a living body, with each of temporal sections of content, in which
   the relative load data is converted into absolute load data, which is the time-series load data absolute to a player who reproduces the content, on the basis of a normalization parameter of the player.
(2) The information processing apparatus according to (1), in which
   the normalization parameter of the player is calculated on the basis of a daily log including exercise data and biological information in daily life of the player.
(3) The information processing apparatus according to (2), in which
   the daily log includes a walking speed and a heart rate.
(4) The information processing apparatus according to any one of (1) to (3), in which
   a load provided to the player is controlled on the basis of the absolute load data in a load providing device worn or used by the player.
(5) The information processing apparatus according to (4), in which
   the absolute load data is adjusted on the basis of a heart rate of the player.
(6) The information processing apparatus according to any one of (1) to (5), further including:
   a display control unit that displays a section timeline panel in which areas corresponding to the respective sections of the content are arranged and a plurality of load icons corresponding to a plurality of types of the relative load data, in which
   the data control unit associates the relative load data with the sections of the content in response to an operation of an operator who arranges the load icons in the areas of the section timeline panel.
(7) The information processing apparatus according to (6), in which
   the operation of arrangement in the areas of the section timeline panel performed by the operator includes drag and drop.
(8) The information processing apparatus according to (6), in which
   the display control unit preferentially displays the load icon corresponding to a content of the content among a plurality of the load icons.
(9) The information processing apparatus according to (6), in which
   the display control unit recommends and displays the load icon corresponding to the section for each of the areas of the section timeline panel.
(10) The information processing apparatus according to (6), in which
   the display control unit displays at least one of the load icon with a color corresponding to a size of the relative load data or the load icon on which an image corresponding to the size of the relative load data is drawn.
(11) The information processing apparatus according to (6), in which
   the display control unit further displays a relative load timeline panel on which a temporal change of the relative load data corresponding to the load icon selected by the operator is graphically displayed.
(12) The information processing apparatus according to (11), in which
   the data control unit edits a graph of the relative load data in response to an operation of the operator performed on the relative load timeline panel.
(13) The information processing apparatus according to (6), in which
   the display control unit further displays the content associated with the relative load data.
(14) The information processing apparatus according to (6), in which
   the display control unit changes display of the load icon corresponding to the relative load data according to the normalization parameter of the operator.
(15) The information processing apparatus according to any one of (1) to (14), in which
   the data control unit adds the relative load data to the content as metadata.
(16) The information processing apparatus according to any one of (1) to (14), in which
   the data control unit associates the relative load data with the content as data different from the content.
(17) The information processing apparatus according to any one of (1) to (16), in which
   the sections of the content are divided at a predetermined time interval.
(18) The information processing apparatus according to any one of (1) to (16), in which
   the sections of the content are divided according to a content of the content.
(19) The information processing apparatus according to any one of (1) to (18), in which
   the normalization parameter includes a maximum oxygen intake.
(20) An information processing method including:
   associating, by an information processing apparatus, relative load data, which is time-series load data relative to a living body, with each of temporal sections of content, in which
   the relative load data is converted into absolute load data, which is the time-series load data absolute to a player who reproduces the content, on the basis of a normalization parameter of the player.
(21) A program for causing a computer to function as:
   a data control unit that associates relative load data, which is time-series load data relative to a living body, with each of temporal sections of content, in which
   the relative load data is converted into absolute load data, which is the time-series load data absolute to a player who reproduces the content, on the basis of a normalization parameter of the player.

### REFERENCE SIGNS LIST

- 1: Relative load calculation device
- 2: Relative load creation/editing device
- 3: Relative load reproduction device
- 4: Normalization parameter calculation device
- 5: Data saving unit
- 11: Absolute load conversion unit
- 12: Relative load conversion unit
- 21: Data control unit
- 22: Display control unit
- 23: Display unit
- 24: Operation unit
- 25: Data storage unit
- 41: Absolute load conversion unit
- 42: Load control unit
- 43: Load adjustment unit
- 61: Data extraction unit
- 62: Representative heart rate DB
- 63: VO2Max estimation unit
- 1001: CPU

## Claims

1. An information processing apparatus comprising:
a data control unit that associates relative load data, which is time-series load data relative to a living body, with each of temporal sections of content, wherein
the relative load data is converted into absolute load data, which is the time-series load data absolute to a player who reproduces the content, on a basis of a normalization parameter of the player.

2. The information processing apparatus according to claim 1, wherein
the normalization parameter of the player is calculated on a basis of a daily log including exercise data and biological information in daily life of the player.

3. The information processing apparatus according to claim 2, wherein
the daily log includes a walking speed and a heart rate.

4. The information processing apparatus according to claim 1, wherein
a load provided to the player is controlled on a basis of the absolute load data in a load providing device worn or used by the player.

5. The information processing apparatus according to claim 4, wherein
the absolute load data is adjusted on a basis of a heart rate of the player.

6. The information processing apparatus according to claim 1, further comprising:
a display control unit that displays a section timeline panel in which areas corresponding to the respective sections of the content are arranged and a plurality of load icons corresponding to a plurality of types of the relative load data, wherein
the data control unit associates the relative load data with the sections of the content in response to an operation of an operator who arranges the load icons in the areas of the section timeline panel.

7. The information processing apparatus according to claim 6, wherein
the display control unit preferentially displays the load icon corresponding to a content of the content among a plurality of the load icons.

8. The information processing apparatus according to claim 6, wherein
the display control unit recommends and displays the load icon corresponding to the section for each of the areas of the section timeline panel.

9. The information processing apparatus according to claim 6, wherein
the display control unit displays at least one of the load icon with a color corresponding to a size of the relative load data or the load icon on which an image corresponding to the size of the relative load data is drawn.

10. The information processing apparatus according to claim 6, wherein
the display control unit further displays a relative load timeline panel on which a temporal change of the relative load data corresponding to the load icon selected by the operator is graphically displayed.

11. The information processing apparatus according to claim 10, wherein
the data control unit edits a graph of the relative load data in response to an operation of the operator performed on the relative load timeline panel.

12. The information processing apparatus according to claim 6, wherein
the display control unit further displays the content associated with the relative load data.

13. The information processing apparatus according to claim 6, wherein
the display control unit changes display of the load icon corresponding to the relative load data according to the normalization parameter of the operator.

14. The information processing apparatus according to claim 1, wherein
the data control unit adds the relative load data to the content as metadata.

15. The information processing apparatus according to claim 1, wherein
the data control unit associates the relative load data with the content as data different from the content.

16. The information processing apparatus according to claim 1, wherein
the sections of the content are divided at a predetermined time interval.

17. The information processing apparatus according to claim 1, wherein
the sections of the content are divided according to a content of the content.

18. The information processing apparatus according to claim 1, wherein
the normalization parameter includes a maximum oxygen intake.

19. An information processing method comprising:
associating, by an information processing apparatus, relative load data, which is time-series load data relative to a living body, with each of temporal sections of content, wherein
the relative load data is converted into absolute load data, which is the time-series load data absolute to a player who reproduces the content, on a basis of a normalization parameter of the player.

20. A program for causing a computer to function as:
a data control unit that associates relative load data, which is time-series load data relative to a living body, with each of temporal sections of content, wherein
the relative load data is converted into absolute load data, which is the time-series load data absolute to a player who reproduces the content, on a basis of a normalization parameter of the player.
